Europäisches Patentamt

European Patent Office

Office européen des brevets

(11) Veröffentlichungsnummer : **0 061 738**
**B1**

(12) **EUROPÄISCHE PATENTSCHRIFT**

(45) Veröffentlichungstag der Patentschrift :
07.05.86

(51) Int. Cl.⁴ : **C 13 K 13/00**, A 61 K 31/70

(21) Anmeldenummer : **82102537.6**

(22) Anmeldetag : **26.03.82**

(54) **Verfahren zur Herstellung eines hohen Anteil an Isomaltopentaose und Isomaltohexaose aufweisenden Gemisches.**

(30) Priorität : **01.04.81 DE 3113035**

(43) Veröffentlichungstag der Anmeldung :
**06.10.82 Patentblatt 82/40**

(45) Bekanntmachung des Hinweises auf die Patenterteilung : **07.05.86 Patentblatt 86/19**

(84) Benannte Vertragsstaaten :
**AT BE CH DE FR GB IT LI LU NL SE**

(56) Entgegenhaltungen :
**DE-A- 2 815 967**
**JOURNAL FÜR PRAKTISCHE CHEMIE, Band 313, Heft 5, 1971, Seiten 861-874, Leipzig, CH. B. TEICHMANN: "Gewinnung von Zuckern der Isomaltosereihe (Isomaltose bis Isomaltododekaose) durch Partialhydrolyse von Dextran" Seiten 865-870**

(73) Patentinhaber : **Laboratorien Hausmann AG**
**Rechenstrasse 37**
**CH-9001 St. Gallen (CH)**

(72) Erfinder : **Geisser, Peter, Dr.rer.nat.**
**Haselstrasse 12**
**CH-9013 St. Gallen (CH)**

(74) Vertreter : **Türk, Dietmar, Dr. rer. nat. et al**
**Redies, Redies, Türk & Gille Patentanwälte Brucknerstrasse 20**
**D-4000 Düsseldorf 13 (DE)**

**Beschreibung**

Die vorliegende Erfindung betrifft ein Verfahren zur Herstellung eines einen hohen Anteil an Isomaltopentaose und Isomaltohexaose aufweisenden Gemisches von Isomalto-Oligosacchariden durch partielle Hydrolyse von Dextran mit einem mittleren Molekulargewicht $\bar{M}_w$ von 12 000 bis 22 000, das in einer Menge von 25 bis 45 g in 100 g Wasser gelöst, ist, mittels Salzsäure und Abtrennung eines Teils der entstandenen Glucose, Isomaltose und der anderen niedrigmolekularen Zucker sowie der anorganischen Bestandteile. Das erfindungsgemäß hergestellte Gemisch kann in sterilen wäßrigen Lösungen, die dieses Gemisch enthalten, zur parenteralen Verabreichung verwendet werden.

Als Plasmaersatzstoffe zur parenteralen Verabreichung werden u. a. in großem Umfang Lösungen von sogenanntem klinischem Dextran verwendet. (Angew. Chem. 1968, Seite 719 bis 725). Derartige klinische Dextrane haben im allgemeinen Molekulargewichte $\bar{M}_w$ im Bereich von 30 000 bis 80 000. Bei Verwendung von klinischen Dextranlösungen kann es in manchen Fällen (etwa 3 Fälle pro 10 000) zu folgenden Nebenwirkungen kommen : Hautausschläge, Fieber, Tachykarchie, Hypotonie, Erbrechen, Schockzustände, Herz- und Atmungsstillstand (J. Ring, K. Messmer, The Lancet, 1977, p. 466).

Es ist weiterhin bekannt, daß diese Nebenwirkungen weitgehend oder vollständig vermieden werden können, wenn vor oder gleichzeitig mit der Verabreichung von klinischem Dextran Isomaltopentaose und/oder Isomaltohexaose oder diese Isomalto-Oligosaccharide enthaltende Gemische parenteral verabreicht werden. Die Inhibitorwirkung von Oligoisomaltosen sowie von deren Gemischen auf die Polyisomaltose (Dextran)-Antikörper-Präzipitaion mit klinischen Dextranen wurde von verschiedenen Autoren ausführlich beschrieben (vergl. E.A. Kabat and A.E. Bezer, Arch. Biochem. and Biophys. *78*, 306-318 (1958) ; E.A. Kabat, Fed. Prod. *21*, 694 (1962) ; E.A. Kabat and M.M. Mayer, Experimental Immunochemistry, 2nd Ed. 1961, C.C. Thomas (Publisher) p. 241-267. Aus diesen Arbeiten ist ersichtlich, daß die optimale Inhibitorwirkung mit Isomaltopentaose und Isomaltohexaose sowie eventuell mit Isomaltotetraose und Isomaltoheptaose erreicht werden kann. Glucose, Isomaltose sowie Isomaltotriose haben praktisch keine Wirkung.

Da die Isomaltopentaose in reiner Form sehr schwer und in wirtschaftlich nicht vertretbarer Weise hergestellt werden können, ist es bekannt, für diesen Zweck Oligoisomaltosegemische einzusetzen, die durch partielle Hydrolyse von Dextran mit Mineralsäure erhalten wurden, vergl. W. Richter, Int. Arch. Allergy *41*, 826-844 (1972) ; W. Richter, Int. Arch. Allergy *41*, 930-936 (1973) ; B. Teichmann, K. Himmelspach und O. Westphal, J. prakt. Chem. *313*, 861-874 (1971) ; DE-A-2 815 967. Aus der vorstehend erwähnten Arbeit von Teichmann ist es bekannt, daß durch spezielle Steuerung der Hydrolysebedingungen und durch Fraktionierung des Hydrolysegemisches möglichst hohe Ausbeuten speziell an Isomaltotetraose bis Isomaltooctaose erhalten werden können. Diese bekannten Verfahren, insbesondere der DE-A-2 815 967, haben jedoch den Nachteil, daß eine außerordentlich lange Hydrolysezeit erforderlich ist und die Fraktionierung des Hydrolysegemisches schwierig ist. Es müssen wiederholte, mindestens dreimalige, Fällungen mit Äthanol durchgeführt werden, um Gemische von Isomalto-Oligosacchariden mit einem ausreichend hohen Anteil an Isomaltopentaose und Isomaltohexaose zu erhalten. Die Ausbeuten an derartigen Isomalto-Oligosacchariden sind deshalb gering und betragen gemäß DE-A-2 815 967 nur etwa 25 bis 38 Gew.-%, bezogen auf das eingesetzte Dextran, wobei die Ausbeute an Isomaltopentaose und Isomaltohexaose, die in dem isolierten Gemisch enthalten ist, zusammen nur etwa 7 bis 11 Gew.-%, bezogen auf das eingesetzte Dextran, betragen.

Der vorliegenden Erfindung liegt die Aufgabenstellung zugrunde, ein Verfahren zur Herstellung eines hohen Anteil an Isomaltopentaose und Isomaltohexaose aufweisenden Gemisches von Isomalto-Oligosacchariden zu finden, das in technisch einfacherer Weise durchgeführt werden kann, wobei insbesondere die Hydrolysezeit und die Aufarbeitung verkürzt werden.

Es wurde nun gefunden, daß diese Aufgabenstellung überraschenderweise dadurch gelöst werden kann, daß das Hydrolyseverfahren in der nachfolgend definierten Weise durchgeführt wird.

Gegenstand der vorliegenden Erfindung ist demgemäß ein Verfahren zur Herstellung eines einen hohen Anteil an Isomaltopentaose und Isomaltohexaose aufweisenden Gemisches von Isomalto-Oligosacchariden durch partielle Hydrolyse von Dextran mit einem mittleren Molekulargewicht $\bar{M}_w$ von 12 000 bis 22 000, das in einer Menge von 25 bis 45 g in 100 g Wasser gelöst, ist, mittels Salzsäure und Abtrennung eines Teils der entstandenen Glucose, Isomaltose und der anderen niedrigmolekularen Zucker sowie der anorganischen Bestandteile, das dadurch gekennzeichnet ist, daß man

a) zur Hydrolyse Chlorwasserstoff in einer Menge von 0.9 g bis 1.3 g HCl pro 100 g Wasser einsetzt,

b) die Hydrolyse bei 95 °C bis zur Rückflußtemperatur für eine Dauer von 40 bis 250 Minuten durchführt,

c) die Abtrennung eines Teils der entstandenen Glucose, Isomaltose und der anderen niedrigmolekularen Zucker sowie der anorganischen Bestandteile durch eine Fällung mit Äthanol und Gewinnung des Niederschlags vornimmt, wobei die zu fällende Lösung eine Kohlenhydratkonzentration von weniger als 30 g/100 ml Lösung aufweist und man mindestens 80 g/g %igen Äthanol in einer solchen Menge zusetzt, daß 100 g Mischung 57 bis 70 g Äthanol enthalten, oder daß man die Abtrennung eines Teils der entstandenen Glucose, Isomaltose und der anderen niedrigmolekularen Zucker sowie der anorganischen Bestandteile mittels Mischungen aus stark sauren und stark basischen Ionenaustauscherharzen vornimmt und das einen hohen Anteil an Isomaltopentaose und Isomaltohexaose aufweisende Gemisch aus den ersten mit Wasser erhaltenen Eluatfraktionen gewinnt.

Es ist überraschend, daß trotz der angewandten hohen Temperaturen für die Hydrolyse und der hohen Säurekonzentration, d. h. verhältnismäßig scharfe Hydrolysebedingungen, eine partielle Hydrolyse im Sinne der gewünschten Aufgabenstellung abläuft, wobei Isomaltopentaose und Isomaltohexaose in hoher Ausbeute erhalten wird. Bezogen auf das eingesetzte Dextran wird gemäß dem Verfahren der Erfindung eine Ausbeute an Isomaltopentaose und Isomaltohexaose zusammen bei nur einmaliger Äthanolfällung, als in einfacherer Weise, von über 12 % erzielt; bei der Ionenaustauscherchromatographie sogar eine Ausbeute von 16 % bis zu 25 %.

Unter einen hohen Anteil an Isomaltopentaose und Isomaltohexaose aufweisenden Gemischen werden gemäß der Erfindung solche Gemische verstanden, die einen möglichst hohen Anteil an den sogenannten Oligosacchariden aufweisen, wobei ein hoher Anteil schon dann vorliegt, wenn Isomaltopentaose und Isomaltohexaose zusammen einen Anteil von mehr als 11 Gew.-%, vorzugsweise mehr als 12 Gew.-% und besonders bevorzugt mehr als 16 Gew.-% im Gemisch vorliegen. Wie aus dem Stand der Technik bekannt, sind die anderen in der Lösung enthaltenen Isomalto-Oligosaccharide als Hapten weniger oder überhaupt nicht wirksam. Ihre Gegenwart wird aber in Kauf genommen, weil sie nicht schädlich ist, und weil es nach dem Stand der Technik eine beträchtlichen wirtschaftlichen Aufwand darstellen würde, die Isomaltopentaose und Isomaltohexaose stärker anzureichern.

Als Ausgangsprodukt für das Verfahren gemäß der Erfindung können die nach dem Stand der Technik zur Hapten-Herstellung eingesetzten Dextrane verwendet werden, wobei wie nach dem Stand der Technik, teilweise abgebautes Dextran mit einem $\bar{M}_w$ im Bereich von 12 000 bis 22 000, insbesondere im Bereich von 16 000 bis 18 000 bevorzugt ist, wobei diese Dextranfraktion in bekannter Weise zweckmäßig erhalten wurde aus Dextran von dem Mikroorganismus NRRL B-512.

Zweckmäßig wird das Dextran gemäß der Erfindung in hoher Konzentration gelöst in Wasser hydrolysiert, wobei die Lösung zweckmäßig mindestens etwa 30, vorzugsweise mindestens etwa 33 g und zweckmäßig höchstens etwa 40, vorzugsweise höchstens etwa 38 g, gelöst in 100 g Wasser, enthält.

Die Menge an Chlorwasserstoff beträgt mindestens etwa 0.9 g, vorzugsweise mindestens 1.0 g, und höchstens etwa 1.3 g, vorzugsweise höchstens etwa 1.2 g pro 100 g Wasser. Die Hydrolyse wird bei möglichst hoher Temperatur durchgeführt, zweckmäßig bei 98 °C bis zur Rückflußtemperatur. Die optimale Hydrolysedauer bei der genannten Temperatur liegt bei mindestens etwa 60 Minuten, vorzugsweise mindestens etwa 80 Minuten, und zweckmäßig höchstens etwa 170 Minuten, vorzugsweise höchstens etwa 125 Minuten.

Ein besonderer Vorteil des Verfahrens der Erfindung liegt darin, daß die Fraktionierung, d. h. die Abtrennung eines Teils der entstandenen Glucose, Isomaltose und der anderen niedrigmolekularen Zucker sowie der anorganischen Bestandteile außerordentlich einfach ist. Durch nur eine einzige Fällung mittels Äthanol können diese Bestandteile in ausreichendem Maße abgetrennt werden. Sie verbleiben in der Lösung, während das gewünschte Gemisch ausgefällt wird. Dieses kann abgetrennt und getrocknet werden. Zur Verwendung kann es dann wieder gelöst werden. Es ist außerordentlich überraschend, daß eine einzige Fällung mit Äthanol ausreichend ist, da nach dem Stand der Technik eine wiederholte Fällung durchgeführt werden muß.

Zweckmäßig wird die Fällung mit Äthanol von mindestens 80 % g/g so durchgeführt, daß eine Mischung mit einem Gewichtsanteil an Äthanol von 61 bis 66 g Äthanol resultiert.

Der Ausdruck « g/g % » bedeutet den prozentuellen Anteil in Gewicht pro Gewicht.

Durch die Ionenaustauscherchromatographie können größtenteils die unerwünschten Bestandteile entfernt werden. Es war dabei vielleicht in gewissem Umfang zu erwarten, daß die selbst durch Äthanolfällung nicht abtrennbaren höhermolekularen Bestandteile abgetrennt werden können. Es ist aber außerordentlich überraschend, daß neben der bekannten Abtrennung der anorganischen Salze... vor allem Glucose, Maltose und Maltotriose, d. h. die niedrigmolekularen Kohlenhydrate, durch Ionenaustauscherchromatographie in außerordentlich einfacher Weise größtenteils entfernt werden können.

Als Ionenaustauscher sind Mischungen aus stark sauren und stark basischen Ionenaustauscherharzen geeignet, z. B. eine Mischung von Dowex 50 W mit Dowex 1 (Warenzeichen der Firma The Dow Chemical Co., USA). Es konnen auch sogenannte Mischbettaustauscher eingesetzt werden, wie der Ionenaustauscher V (LAB) der Firma Merck, Darmstadt, Bundesrepublik Deutschland.

Die dem Ionenaustauscherharz zugeführte Lösung hat zweckmäßig einen Kohlenhydratgehalt von 5 bis 15 g/100 ml Lösung, ist also relativ verdünnt. Die Menge der Eluatfraktionen, aus denen das einen hohen Anteil an Isomaltopentaose und Isomaltohexaose aufweisende Gemisch gemäß der Erfindung gewonnen wird. beträgt zweckmäßig 100 bis 400 %, vorzugsweise 200 bis 300 %, der Menge der dem Ionenaustauscherharz zugeführten Kohlenhydratlösung.

Wie oben bereits ausgeführt, ist der Gehalt an Kohlenhydraten in den Lösungen, die der Äthanolfällung unterworfen werden, höher. Vorzugsweise liegt der Kohlenhydratgehalt dabei im Bereich von etwa 21 bis etwa 29 g/100 ml Lösung.

Die Hydrolyselösungen werden durch Zugabe von Wasser oder wäßrigen Lösungen, die zur Neutralisation eingesetzt werden, auf den gewünschten Kohlenhydrat-Gehalt verdünnt. Es ist ganz außerordentlich überraschend, daß durch die niedrigeren Kohlenhydrat-Gehalte eine derart einfache

3

Trennung ermöglich wird. Gemäß der oben erwähnten DE-A-2 815 967 werden dagegen Lösungen der Äthanolfällung unterworfen, die im Beispiel 1A 40 g/100 ml Lösung und in den Beispielen 1B und 1C 60 g/100 ml Lösung Kohlenhydrate enthalten. Offenbar hat man angenommen, daß hohe Konzentrationen für das Verfahren günstig sind, was überraschenderweise jedoch nicht zutrifft.

Bei Fraktionierung mittels Ionenaustauscherchromatographie können die erhaltenen Lösungen unmittelbar oder gegebenenfalls nach Einengen oder Verdünnen auf die gewünschte Konzentration für Infusionszwecke eingesetzt werden. Falls erforderlich, können die Lösungen durch Filtration, z. B. über Aktivkohle gereinigt werden.

Zweckmäßig wird die Fraktionierung mit der abgekühlten und im wesentlichen neutralisierten Hydrolyselösung durchgeführt, wie dies aus dem Stand der Technik bekannt ist.

Das erfindungsgemäß hergestellte Gemisch kann gegebenenfalls im Gemisch mit klinischem Dextran und/oder üblichen Zusatzstoffen, in steriler wäßriger Lösung zur parenteralen Verabreichung verwendet werden. Durch das erfindungsgemäße Verfahren wird es somit möglich eine sterile wäßrige Lösung zur parenteralen Verabreichung, enthaltend einen hohen Anteil an Isomaltopentaose und Isomaltohexaose aufweisendes Gemisch von Isomalto-Oligosacchariden und gegebenenfalls klinisches Dextran als Plasmaersatzmittel und/oder übliche Zusatzstoffe, bereitzustellen.

Die sterile wäßrige Lösung, enthaltend das gemäß dem Verfahren der Erfindung erhältliche Gemisch kann, wie dies bekannt ist, vor oder zusammen mit dem klinischen Dextran verabreicht werden. Übliche Zusatzstoffe der sterilen wäßrigen Lösung sind NaCl, KCl, $NaOOCCH_3$, $CaCl_2$ und $MgCl_2$. Die Sterilisation erfolgt in an sich bekannter Weise.

## Beispiel 1

22 kg Dextran erhalten aus Dextran mit dem Mikroorganismus NRRL B-512, mit einem mittleren Molekulargewicht $\bar{M}_w$ von 17 000 werden unter gutem Rühren bei 40 °C in 64 l Wasser für Infusionszwecke gelöst und die Lösung anschließend auf Rückflußtemperatur erhitzt. Nun werden innerhalb 15 Sekunden 3.16 l (3 550 g) 6.15 molare Salzsäure in einem Guß zugegeben und während 100 Minuten weiter am Rückfluß erhitzt. Nach 100 Minuten Hydrolysezeit wird innerhalb von 10 Minuten auf 25 °C abgekühlt und anschließend mit etwa 2 l einer Natronlauge von 40 g/100 ml ein pH von 5.5 bis 6.5 eingestellt. Das Reaktionsgemisch von 78 l (87 kg) wird nun mit 203 kg 88.6 (g/g) %igem Äthanol versetzt, und die Ausfällung wird während 2 bis 5 Stunden absetzen gelassen. Nach dem Absaugen der obenstehenden Lösung wird der Rückstand bei 40 °C und 15 mbar getrocknet. Die Ausbeute beträgt 10.9 kg. In diesem Beispiel betrug die Menge an Kohlenhydraten in der Lösung vor Zugabe des Äthanols 28.2 g/100 ml Lösung. Nach der Zugabe des Äthanols betrug der Gehalt an Äthanol in der Gesamtmischung 62 g/100 g.

In der nachfolgenden Tabelle 1 sind die Analysenergebnisse zusammengestellt. In der Spalte « Ausbeute » ist die Ausbeute des jeweiligen Saccharids mit dem in Spalte 1 angegebenen Oligolimerisierungsgrad, bezogen auf das eingesetzte Dextran angegeben.

In der Spalte « Verteilung » ist der prozentuale Anteil des jeweiligen Saccharids in der abgetrennten Mischung gemäß der Erfindung angegeben, d. h. in dem einen hohen Anteil an Isomaltopentaose und an Isomaltohexaose aufweisenden Gemisch.

Die Tabelle 1 enthält auch die entsprechenden Werte für die Beispiele 2, 3, 4 und 11.

In Tabelle 2 sind die entsprechenden Werte gemäß DE-A-2 815 967 zusammengestellt. Durch Vergleich der Tabellen ergibt sich, daß die Ausbeuten gemäß der Erfindung beträchtlich überlegen sind denjenigen der DE-A-2 815 967. In den meisten Beispielen gemäß der Erfindung ist auch der Anteil der Polysaccharide mit dem Oligomerisierungsgrad 5 und 6 (vergl. Spalte : Verteilung) höher als gemäß der DE-A-2 815 967. Bei denjenigen Beispielen gemäß der Erfindung, bei denen dieser Anteil etwas geringer ist, überwiegt die beträchtlich höhere Ausbeute.

## Beispiel 2

Die Hydrolyse wird wie unter Beispiel 1 durchgeführt, jedoch mit 236 kg Äthanol von 88,6 (g/g) % gefällt. Die Ausbeute beträgt 11.9 kg. Die Kohlenhydratkonzentration von der Fällung betrug 28.2 g/100 ml. Nach der Fällung lag das Äthanol in der Mischung in einer Menge von 65 g/100 g vor.

## Beispiel 3

Die Hydrolyse wird wie unter Beispiel 1 durchgeführt, jedoch die Lösung vor dem Fällen mit Wasser für Infusionszwecke im Verhältnis 4 : 1 verdünnen. Die Fällung erfolgt mit 296 kg Äthanol von 88.6 (g/g) %. Die Ausbeute beträgt 9.5 kg 22.6 g/100 ml. Nach der Fällung lag das Äthanol in der Mischung in einer Menge von 65 g/100 g vor.

## Beispiel 4

4.4 kg Dextran mit einem mittleren Molekulargewicht $\bar{M}_w$ von 17 000 werden unter Rühren bei 40 °C

in 12.8 l Wasser für Infusionszwecke gelöst und die Lösung anschließend am Rückfluß erhitzt. Nun werden innerhalb 15 Sekunden 632 ml (710 g) 6.15 molare Salzsäure in einem Guß zugegeben und während 100 Minuten weiter rückflussiert. Nach 100 Minuten Hydrolysezeit wird innerhalb von 10 Minuten auf 25 °C abgekühlt und anschließend mit Natronlauge ein pH von 5.5 bis 6.5 eingestellt. (Diese Lösung ist mit der aus Beispiel 1 identisch). Das Reaktionsgemisch wird nun mit Wasser für Infusionszwecke auf 44 l verdünnt und anschließend über eine Ionenaustauschersäule mit 110 kg Mischbettaustaucher der Firma Merck chromatographiert. Die ersten zwei Fraktionen à 44 l enthalten zusammen 2.68 kg Oligoisomaltosegemisch der in Tabelle 1 angegebenen Zusammensetzung. Nach dem Abdestillieren von Wasser auf ein Volumen von 17.8 l erhält man eine 15 %ige Oligoisomaltoselösung, welche direkt nach einer Aktivkohlefiltration in Stechampullen abgefüllt und anschließend sterilisiert werden kann.

## Beispiel 5

Die Hydrolyse und Ionenaustauscherchromatographie wird wie in Beispiel 4 durchgeführt. Anstelle des Abdestillierens von Wasser wird die Lösung von 88 l, enthaltend 2.68 kg Oligoisomaltosegemisch, auf 40 °C erwärmt und mit 26.8 kg Dextran 60 versetzt und nach Zugabe der gewünschten Elektrolyte (NaCl, KCl, KOOCCH$_3$, NaOOCCH$_3$, CaCl$_2$, MgCl$_2$) oder Kohlehydrate (Sorbit) mit Wasser für Infusionszwecke auf 447 l verdünnt, über Aktivkohle filtiert und anschließend nach dem Abfüllen in Infusionsbehälter hitzesterilisiert.

## Beispiel 6

Gleiches Vorgehen wie Beispiel 5. Jedoch Einsatz von Dextran 70.

## Beispiel 7

Gleiches Vorgehen wie Beispiel 5. Jedoch Einsatz von 44.7 kg Dextran 40.

## Beispiel 8

Gleiches Vorgehen wie Beispiel 5. Jedoch Einsatz von 53.6 kg Dextran 60 und Verdünnen auf ein Endvolumen von 893 l.

## Beispiel 9

Gleiches Vorgehen wie Beispiel 5. Jedoch Einsatz von 53.6 kg Dextran 70 und Verdünnen auf ein Endvolumen von 893 l.

## Beispiel 10

Gleiches Vorgehen wie Beispiel 5. Jedoch Einsatz von 89.3 kg Dextran 40 und Verdünnen auf ein Endvolumen von 893 l.

## Beispiel 11

Die Hydrolyse wird wie in Beispiel 4 ausgeführt, jedoch nur während 80 Minuten am Rückfluß gekocht. Die Chromatographie über die Ionenaustauschersäule wird gleich durchgeführt, jedoch 3 Fraktionen à 44 l gesammelt, welche zusammen 3.82 kg Oligoisomaltosegemisch (Zusammensetzung siehe Tabelle 1) enthalten.

## Beispiel 12

Die Hydrolyse und Ionenaustauscherchromatographie wird wie in Beispiel 11 ausgeführt. Anstelle des Abdestillierens von Wasser wird die Lösung von 132 l, enthaltend 3.82 kg Oligoisomaltosegemisch, auf 40 °C erwärmt und mit 38.2 kg Dextran 60 versetzt und nach Zugabe der gewünschten Elektrolyte (NaCl, KCl, KOOCCH$_3$, NaOOCCH$_3$, CaCl$_2$, MgCl$_2$) oder Kohlehydrat (Sorbit) mit Wasser für Infusionszwecke auf 637 l verdünnt, über Aktivkohle filtiert und anschließend nach dem Abfüllen in Infusionsbehälter hitzesterilisiert.

## Beispiel 13

Gleiches Vorgehen wie Beispiel 12. Jedoch Einsatz von Dextran 70.

## Beispiel 14

Gleiches Vorgehen wie Beispiel 12. Jedoch Einsatz von 63.7 kg Dextran 40.

**0.061 738**

Beispiel 15

Gleiches Vorgehen wie Beispiel 12. Jedoch Einsatz von 76.4 kg Dextran 60 und Verdünnen auf ein Endvolumen von 1 273 l.

Beispiel 16

Gleiches Vorgehen wie Beispiel 12. Jedoch Einsatz von 76.4 kg Dextran 70 und Verdünnen auf ein Endvolumen von 1 273 l.

Beispiel 17

Gleiches Vorgehen wie Beispiel 12. Jedoch Einsatz von 127.3 kg Dextran 40 und Verdünnen auf ein Endvolumen von 1 273 l.

(Siehe Tabelle I Seite 7 f.)

Tabelle 1

| Oligomeri-sierungs-grad | BEISPIEL 1 | | BEISPIEL 2 | | BEISPIEL 3 | | BEISPIEL 4 | | BEISPIEL 11 | |
|---|---|---|---|---|---|---|---|---|---|---|
| | Ver-teilung $[\%]$ | Aus-beute $[\%]$ | Ver-teilung $[\%]$ | Aus-beute $[\%]$ | Ver-teilung $[\%]$ | Aus-beute $[\%]$ | Ver-teilung $[\%]$ | Aus-beute $[\%]$ | Ver-teilung $[\%]$ | Aus-beute $[\%]$ |
| 1 | 4.9 | 2.4 | 6.0 | 3.2 | 4.3 | 1.9 | 3.6 | 2.2 | 2.8 | 2.4 |
| 2 | 7.5 | 3.7 | 7.8 | 4.2 | 6.9 | 3.0 | 7.8 | 4.7 | 7.8 | 6.8 |
| 3 | 9.0 | 4.5 | 9.4 | 5.1 | 8.9 | 3.8 | 12.2 | 7.4 | 12.7 | 11.0 |
| 4 | 10.6 | 5.3 | 10.7 | 5.8 | 10.4 | 4.5 | 14.7 | 9.0 | 14.7 | 12.8 |
| 5 | 12.8 | 6.4 | 11.9 | 6.4 | 13.2 | 5.7 | 14.4 | 8.8 | 15.2 | 13.2 |
| 6 | 12.8 | 6.4 | 11.5 | 6.2 | 16.2 | 7.0 | 13.2 | 8.0 | 13.6 | 11.8 |
| 7 | 10.5 | 5.2 | 9.8 | 5.3 | 12.5 | 5.4 | 9.5 | 5.8 | 10.6 | 9.2 |
| 8 | 9.1 | 4.5 | 9.7 | 5.2 | 8.8 | 3.8 | 7.8 | 4.7 | 8.6 | 7.5 |
| 9 | 9.0 | 4.5 | 10.4 | 5.6 | 6.5 | 2.8 | 6.5 | 4.0 | 6.0 | 5.2 |
| 10 11 12 13 14 | 13.8 | 6.9 | 12.8 | 6.9 | 12.3 | 5.3 | 10.3 | 6.3 | 8.0 | 6.9 |
| 15 - 20 | -- | -- | -- | -- | -- | -- | -- | -- | -- | -- |
| Summe | 100.0 | 49.8 | 100.0 | 53.9 | 100.0 | 43.2 | 100.0 | 60.9 | 100.0 | 86.8 |

Tabelle 2 (DE-OS 2 815 967)

| Oligomerisie-rungsgrad | Verteilung % | Ausbeute Beispiel 1 A % | Ausbeute Beispiel 1 B, 1 C % | Ausbeute Beispiel 1 D % |
|---|---|---|---|---|
| 1 | 2.9 | 0.73 | 1.09 | 0.94 |
| 2 | 6.8 | 1.70 | 2.57 | 2.19 |
| 3 | 10.7 | 2.68 | 4.04 | 3.45 |
| 4 | 14.6 | 3.65 | 5.51 | 4.71 |
| 5 | 15.5 | 3.88 | 5.85 | 5.00 |
| 6 | 13.6 | 3.40 | 5.13 | 4.39 |
| 7 | 10.7 | 2.68 | 4.04 | 3.45 |
| 8 | 6.8 | 1.70 | 2.57 | 2.19 |
| 9 | 4.9 | 1.23 | 1.85 | 1.58 |
| 10 11 12 13 14 | 11.6 | 2.90 | 4.38 | 3.74 |
| 15 – 20 | 1.9 | 0.48 | 0.72 | 0.61 |
| Summe | 100.0 | 25.03 | 37.75 | 32.25 |

**Patentansprüche**

1. Verfahren zur Herstellung eines einen hohen Anteil an Isomaltopentaose und Isomaltohexaose aufweisenden Gemische von Isomalto-Oligosacchariden durch partielle Hydrolyse von Dextran mit einem mittleren Molekulargewicht $\bar{M}_w$ von 12 000 bis 22 000, das in einer Menge von 25 bis 45 g in 100 g Wasser gelöst, ist, mittels Salzsäure und Abtrennung eines Teils der entstandenen Glucose, Isomaltose und der anderen niedrigmolekularen Zucker sowie der anorganischen Bestandteile, dadurch gekennzeichnet, daß man.

a) zur Hydrolyse Chlorwasserstoff in einer Menge von 0.9 g bis 1.3 g HCl pro 100 g Wasser einsetzt,

b) die Hydrolyse bei 95 °C bis zur Rückflußtemperatur für eine Dauer von 40 bis 250 Minuten durchführt,

c) die Abtrennung eines Teils der entstandenen Glucose, Isomaltose und der anderen niedrigmolekularen Zucker sowie der anorganischen Bestandteile durch eine Fällung mit Äthanol und Gewinnung des Niederschlags vornimmt, wobei die zu fällende Lösung eine Kohlenhydratkonzentration von weniger als 30 g/100 ml Lösung aufweist und man mindestens 80 g/g %igen Äthanol in einer solchen Menge zusetzt, daß 100 g Mischung 57 bis 70 g Äthanol enthalten, oder daß man die Abtrennung eines Teils der entstandenen Glucose, Isomaltose und der anderen niedrigmolekularen Zucker sowie der anorganischen Bestandteile mittels Mischungen aus stark sauren und stark basischen Ionenaustauscherharzen vornimmt und das einen hohen Anteil an Isomaltopentaose und Isomaltohexaose aufweisende Gemisch aus den ersten mit Wasser erhaltenen Eluatfraktionen gewinnt.

2. Verfahren nach Anspruch 1, dadurch gekennzeichnet, daß man das Dextran in einer Menge von 30 bis 40, vorzugsweise von 33 bis 38 g gelöst in 100 g Wasser einsetzt.

3. Verfahren nach Anspruch 1 oder 2, dadurch gekennzeichnet, daß man den Chlorwasserstoff in einer Menge von 1.0 g bis 1.2 g pro 100 g Wasser einsetzt.

4. Verfahren nach einem oder mehreren der Ansprüche 1 bis 3, dadurch gekennzeichnet, daß man die Hydrolyse bei 98 °C bis zur Rückflußtemperatur durchführt.

5. Verfahren nach einem oder mehreren der Ansprüche 1 bis 4, dadurch gekennzeichnet, daß man die Hydrolyse während einer Dauer von 60 bis 170 Minuten, vorzugsweise von 85 bis 125 Minuten durchführt.

6. Verfahren nach einem oder mehreren der Ansprüche 1 bis 5, dadurch gekennzeichnet, daß die zu fällende Lösung in Stufe c) eine Kohlenhydratkonzentration von 21 bis 29 g/100 ml Lösung aufweist.

7. Verfahren nach einem oder mehreren der Ansprüche 1 bis 6, dadurch gekennzeichnet, daß Äthanol in einer solchen Menge zugesetzt wird, daß 100 g Mischung 61 bis 66 g Äthanol enthalten.

8. Verfahren nach einem oder mehreren der Ansprüche 1 bis 5, dadurch gekennzeichnet, daß die dem Ionenaustauscherharz zugeführte Lösung einen Kohlenhydratgehalt von 5 bis 15 g/100 ml Lösung aufweist.

9. Verfahren nach Anspruch 8, dadurch gekennzeichnet, daß die Menge der Eluatfraktionen, aus denen das einen hohen Anteil an Isomaltopentaose und an Isomaltohexaose aufweisende Gemisch gewonnen wird, 100 bis 400 % der Menge der dem Ionenaustauscherharz zugeführten Lösung entspricht.

10. Verfahren nach Anspruch 9, dadurch gekennzeichnet, daß die Menge der Eluatfraktion 200 bis 300 % der Menge der zugeführten Kohlenhydratlösung entspricht.

**Claims**

1. A process for preparing a mixture of isomaltooligosaccharides containing a high proportion of isomaltopentose and isomaltohexose by the partial hydrolysis of dextran having an average molecular weight $M_w$ of from 12,000 to 22,000, which is dissolved in a quantity of from 25 to 45 g in 100 g of water, by means of hydrochloric acid and by separating part of the resulting glucose, isomaltose and the other low molecular weight sugars as well as the inorganic constituents, characterised in that,

a) for hydrolysis, hydrogen chloride is used in a quantity of from 0.9 g to 1.3 g of HCl per 100 g of water,

b) the hydrolysis is carried out at 95 °C up to reflux temperature for a duration of from 40 to 250 minutes,

c) the separation of part of the resulting glucose, isomaltose, ant the other low molecular weight sugars, as well as of the inorganic constituents, is effected by precipitation with ethanol and recovering the precipitate, the solution to be precipitated having a carbohydrate concentration of less than 30 g/100 ml of solution and at least 80 wt/wt % ethanol being added in a quantity such that 100 g of mixture contain from 57 to 70 g of ethanol, or the separation of part of the resulting glucose, isomaltose, and the other low molecular weight sugars, as well as of the organic constituents, is effected by mixtures of strongly acidic and strongly basic ion exchange resins and a mixture containing a high proportion of isomaltopentose and isomaltohexose is recovered from the first eluate fractions obtained with water.

2. A process according to claim 1, characterised in that the dextran is used in a quantity of from 30 to 40, preferably from 33 to 38 g dissolved in 100 g of water.

3. A process according to claim 1 or 2, characterised in that the hydrogen chloride is used in a quantity of from 1.0 g to 1.2 g per 100 g of water.

4. A process according to one or more of claims 1 to 3, characterised in that the hydrolysis is carried out at 98 °C up to reflux temperature.

5. A process according to one or more of claims 1 to 4, characterised in that the hydrolysis is carried out for a duration of from 60 to 170 minutes, preferably from 80 to 125 minutes.

6. A process according to one or more of claims 1 to 5, characterised in that the solution to be precipitated in stage c) has a carbohydrate concentration of from 21 to 29 g/100 ml of solution.

7. A process according to one or more of claims 1 to 6, characterised in that ethanol is added in such a quantity that 100 g of mixture contains from 61 to 66 g of ethanol.

8. A process according to one or more of claims 1 to 5, characterised in that the solution added to ion exchange resin has a carbohydrate content of from 5 to 15 g/100 ml of solution.

9. A process according to claim 8, characterised in that the quantity of the eluate fraction, from which the mixture containing a high proportion of isomaltopentose and isomaltohexose is recovered, corresponds to from 100 to 400 % of the quantity of solution added to the ion exchange resin.

10. A process according to claim 9, characterised in that the quantity of the eluate fraction corresponds to from 200 to 300 % of the quantity of the added carbohydrate solution.

**Revendications**

1. Procédé pour la préparation d'un mélange d'isomalto-oligosaccharides présentant une teneur élevée en isomaltopentose et isomaltohexose par hydrolyse partielle de dextran avec un poids moléculaire moyen $M_w$ de 12 000 à 22 000, qui est dissous en quantité de 25 à 35 g dans 100 g d'eau, à l'aide d'acide chlorhydrique, et par séparation d'une partie de glucose, d'isomaltose et des autres sucres de bas poids moléculaire formés ainsi que des constituants inorganiques, caractérisé en ce que

a) on utilise pour l'hydrolyse l'acide chlorhydrique en quantité de 0,9 g à 1,3 g HCl par 100 g d'eau,

b) on réalise l'hydrolyse à 95 °C jusqu'à la température de reflux pendant une durée de 40 à 250 minutes,

c) on effectue la séparation d'une partie de glucose, d'isomaltose et des autres sucres de bas poids moléculaire formés ainsi que des constituants inorganiques par une précipitation à l'éthanol et l'obtention du précipité, la solution à précipiter présentant une concentration en glucides inférieure à 30 g/100 ml de solution et en ajoutant l'éthanol à au moins 80 g/100 g en quantité telle que 100 g de mélange contiennent 57 à 70 g d'éthanol, ou bien on effectue la séparation d'une partie de glucose, d'isomaltose et des autres sucres de bas poids moléculaire ainsi que des constituants inorganiques à l'aide de mélanges de résines échangeuses d'ions fortement acides et fortement basiques et on obtient le mélange présentant une teneur élevée en isomaltopentose et isomaltohexose à partir des premières fractions d'éluat obtenues avec l'eau.

9

2. Procédé selon la revendication 1, caractérisé en ce qu'on utilise le dextran en quantité de 30 à 40, de préférence de 33 à 38 g, dissous dans 100 g d'eau.

3. Procédé selon la revendication 1 ou 2, caractérisé en ce qu'on utilise l'acide chlorhydrique en une quantité de 1,0 g à 1,2 g par 100 g d'eau.

4. Procédé selon l'une ou plusieurs des revendications 1 à 3, caractérisé en ce qu'on effectue l'hydrolyse à 98 °C jusqu'à la température de reflux.

5. Procédé selon l'une ou plusieurs des revendications 1 à 4, caractérisé en ce qu'on effectue l'hydrolyse pendant une durée de 60 à 170 minutes, de préférence de 80 à 125 minutes.

6. Procédé selon l'une ou plusieurs des revendications 1 à 5, caractérisé en ce que la solution à précipiter dans l'étape c) présente une concentration en glucides de 21 à 29 g/100 ml de solution.

7. Procédé selon l'une ou plusieurs des revendications 1 à 6, caractérisé en ce que l'éthanol est ajouté en une quantité telle que 100 g de mélange contiennent 61 à 66 g d'éthanol.

8. Procédé selon l'une ou plusieurs des revendications 1 à 5, caractérisé en ce que la solution ajoutée à la résine échangeuse d'ions présente une teneur en glucides de 5 à 15 g/100 ml de solution.

9. Procédé selon la revendication 8, caractérisé en ce que la quantité des fractions d'éluat, à partir desquelles est obtenu le mélange présentant une teneur élevée en isomaltopentose et en isomaltohexose, correspond de 100 à 400 % de la quantité de solution ajoutée à la résine échangeuse d'ions.

10. Procédé selon la revendication 9, caractérisé en ce que la quantité de la fraction d'éluat correspond de 200 à 300 % de la quantité de la solution de glucides ajoutée.